(19) Europäisches Patentamt
European Patent Office
Office européen des brevets

(11) Numéro de publication : **0 412 901 B1**

(12) # FASCICULE DE BREVET EUROPEEN

(45) Date de publication du fascicule du brevet : **05.05.93 Bulletin 93/18**

(51) Int. Cl.$^5$ : **A61K 31/44,** C07D 211/70

(21) Numéro de dépôt : **90402257.1**

(22) Date de dépôt : **07.08.90**

(54) **Utilisation de trifluorométhyl-phényltétrahydropyridines pour la préparation de médicaments destinés à combattre les troubles de la motricité intestinale.**

(30) Priorité : **07.08.89 FR 8910617**

(43) Date de publication de la demande : **13.02.91 Bulletin 91/07**

(45) Mention de la délivrance du brevet : **05.05.93 Bulletin 93/18**

(84) Etats contractants désignés : **AT BE CH DE DK FR GB IT LI LU NL SE**

(56) Documents cités :
**EP-A- 0 060 176**
**EP-A- 0 101 381**
**FR-A- 86 403**
**GASTROENTEROLOGY, vol. 51, no. 5, part 1, 1966, The Williams & Wilkins Co.; T.G.C.MURRELL et al., pp. 656-663.**
**J. PHARMACOL. EXP. THER., vol. 248, no. 3, mars 1989, The American Society for Pharmacology and Experimental Therapeutics, US; J.ADRIEN et al., pp. 1222-1230.**
**INTERNATIONAL JOURNAL OF OBESITY, vol. 8, suppl. 1, 1984, R.RONCUCCI et al., pp. 103-117.**
**PHARMACOLOGICAL RESEARCH, vol. 22, suppl. 2, 1990; pp. 53, 137.**

(73) Titulaire : **ELF SANOFI**
**32-34, rue Marbeuf**
**F-75008 Paris (FR)**
(84) **BE CH DE DK FR GB LI LU NL SE AT**
Titulaire : **MIDY S.p.A.**
**Via Piranesi 38**
**I-20137 Milano (IT)**
(84) **IT**

(72) Inventeur : **Bianchetti, Alberto**
**Via Corridoni 11**
**I-20122 Milan (IT)**
Inventeur : **Croci, Tiziano**
**Via Giacinti 2**
**Rozzano, Milan (IT)**
Inventeur : **Manara, Luciano**
**Via Novella 2/2**
**Pietra Marazzi, Alessandria (IT)**

(74) Mandataire : **Gillard, Marie-Louise et al**
**Cabinet Beau de Loménie 158, rue de l'Université**
**F-75340 Paris Cédex 07 (FR)**

EP 0 412 901 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne l'utilisation de trifluorométhylphényltétrahydropyridines pour la préparation de médicaments destinés au traitement des troubles de la motricité intestinale et en particulier au traitement de la constipation.

Les brevets européens 60 176 et 101 381 décrivent des trifluorométhylphényltétrahydropyridines N-substituées à action anorexigène.

On a maintenant trouvé que lesdites trifluorométhylphényltétrahydropyridines possèdent une activité procinétique intestinale et qu'elles sont donc utiles pour le traitement des troubles de la motricité intestinale, et en particulier de la constipation.

On a également trouvé que l'action sur les troubles intestinaux et l'action anticonstipante se manifestent à des doses nettement inférieures à celles qui donnent l'anorexie.

On a enfin trouvé que l'activité anticonstipante des mêmes composés n'est pas accompagnée, aux doses anticonstipantes, d'un effet sédatif.

Ainsi, la présente invention concerne l'utilisation de trifluorométhylphényltétrahydropyridines de formule (I) :

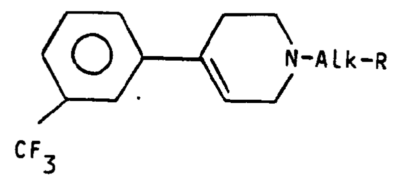

(I)

dans laquelle Alk représente un groupe alkylène à chaîne droite ou ramifiée ayant de 1 à 4 atomes de carbone et R représente un groupe cyano, un groupe acétyle, un groupe cycloalkyle ayant 3 à 7 atomes de carbone, un groupe phényle, un groupe p-tolyle, un groupe pyridyle, un groupe pyridyle 1-oxyde ou un groupe naphtyle, et de leurs sels pharmaceutiquement acceptables, pour la préparation de médicaments destinés à combattre les troubles de la motricité intestinale.

Un groupe de composés I, préférés dans l'utilisation selon la présente invention, comprend les composés de formule (I) dans laquelle R est tel que défini ci-dessus et Alk représente un groupe éthylène dont 1 ou 2 atomes d'hydrogène peuvent être remplacés par un groupe méthyle, ainsi que leurs sels pharmaceutiquement acceptables.

Lorsque, dans la formule (I), R représente le groupe pyridyle, ce groupe est attaché à Alk par un des atomes de carbone en position 2, 3 ou 4 ; lorsque R représente le groupe naphtyle, il est attaché à Alk en position 1 ou en position 2.

Les composés de formule (I) et leur préparation sont décrits dans les documents EP 60 176, EP 101 381 et FR 86 403 (certificat d'addition à FR 1 421 208).

Le composé de formule (I) dans laquelle Alk est un groupe éthylène et R représente un groupe p-tolyle ainsi que ses sels pharmaceutiquement acceptables sont des produits nouveaux et représentent donc avec les compositions pharmaceutiques les contenant d'autres objets spécifiques de la présente invention.

Les principes actifs à utiliser selon la présente invention, soumis à un test au cours duquel on étudie l'élimination fécale chez le rat, ont montré une bonne action procinétique sur l'intestin.

Selon ce test, des rats mâles Crl : CD (Charles River Italia) pesant entre 220 et 250 g sont placés à 8 h du matin, sans nourriture, mais avec libre accès à l'eau, dans des cages individuelles ayant le fond en filet et un plateau situé au-dessous.

A 11 h 30, les produits à l'étude sont administrés par voie orale ou sous-cutanée.

Le schéma de traitement est planifié en utilisant des tableaux de randomisation et 8 animaux par groupe.

Après le traitement, l'ampoule rectale est vidée manuellement des selles éventuellement présentes et les rats sont remis dans leurs cages respectives. Les boulettes sont ramassées, comptées et pesées pour la détermination du poids frais, 90 min après l'administration du produit par voie sous-cutanée ou bien 210 min après l'administration par voie orale. Les selles sont placées dans une étuve et séchées pendant 10 h à 40°C pour la détermination du poids sec ; après 5-6 h, le poids ne varie plus et les selles contiennent approximativement le même pourcentage d'humidité résiduelle.

Le paramètre utilisé pour le calcul de l'activité est le poids sec en grammes des selles réunies cumulativement pendant les 90 min (voie sous-cutanée) ou les 210 min (voie orale) qui suivent l'administration du produit. L'analyse statistique des données est effectuée selon le "Duncan new multiple test".

L'activité relative des composés est calculée sur la base d'un index d'activité (IA-1 g) qui représente la

dose active qui stimule l'élimination de 1 g de selles (poids sec) ; cet index est calculé sur la droite de régression du log-dose/effet (p>0,05).

Chez les témoins qui reçoivent seulement le véhicule, on n'a presque aucune élimination fécale dans la même période. Chez les animaux traités avec des doses élevées, on peut avoir une élimination maximale variable de 12-16 boulettes de selles ayant un poids sec d'environ 1,4-1,8 g. Au-dessus de ces valeurs d'élimination totale, on a l'apparition d'une nette diarrhée qui empêche une quantification exacte.

Dans le tableau qui suit est donné l'IA-1 g de quelques composés de formule (I), en particulier :

- le chlorhydrate de 4-(3-trifluorométhylphényl)-1-(2-cyanoéthyl)-1,2,3,6-tétrahydropyridine (Composé A) ;
- le chlorhydrate de 1-[2-(2-naphtyl)éthyl]-4-(3-trifluorométhylphényl)-1,2,3,6-tétrahydropyridine (Composé B) ;
- le chlorhydrate de 1-[2-(1-naphtyl)éthyl]-4-(3-trifluorométhylphényl)-1,2,3,6-tétrahydropyridine (Composé C) ;
- le chlorhydrate de 1-(2-phényléthyl)-4-(3-trifluorométhylphényl)-1,2,3,6-tétrahydropyridine (Composé D) ;
- le dichlorhydrate de 1-[2-(2-pyridyl)éthyl]-4-(3-trifluorométhylphényl)-1,2,3,6-tétrahydropyridine (Composé E) ;
- le dichlorhydrate de 1-[2-(3-pyridyl)éthyl]-4-(3-trifluorométhylphényl)-1,2,3,6-tétrahydropyridine monohydraté (Composé F) ;
- le dichlorhydrate de 1-[2-(4-pyridyl)éthyl]-4-(3-trifluorométhylphényl)-1,2,3,6-tétrahydropyridine monohydraté (Composé G) ;
- le chlorhydrate de 1-[2-(4-tolyl)éthyl]-4-(3-trifluorométhylphényl)-1,2,3,6-tétrahydropyridine (Composé H).

Ces composés, leurs bases libre et les autres sels pharmaceutiquement acceptables desdites bases libres, représentent des principes actifs préférés pour l'utilisation selon la présente invention.

| | Excrétion de selles chez le rat IA-1 g (mg/kg) | |
|---|---|---|
| | s.c. | os |
| Composé A | n.d. | 16 |
| Composé B | n.d. | 1,3 |
| Composé C | 6 | 3,9 |
| Composé D | 4 | 13 |
| Composé E | 2 | 1,7 |
| Composé F | 1 | 0,6 |
| Composé G | 1 | 5 |
| Composé H | 3 | 2,3 |
| n.d. = non déterminé | | |

De ce tableau, il ressort que les produits de formule (I) sont actifs sur la constipation à des doses très faibles. Les composés F, B, E, H et C se sont montrés très actifs et représentent des principes actifs particulièrement avantageux, de même que les bases libres correspondantes et leurs autres sels pharmaceutiquement acceptables.

L'administration des composés de formule (I) et de leurs sels pharmaceutiquement acceptables peut être effectuée par voie orale, sublinguale, transdermique ou par voie parentérale. La quantité de principe actif à administrer pour traiter les troubles de la motricité intestinale dépend, comme il est habituel, du type de traitement, prophylactique ou thérapeutique, de la nature et de la sévérité des troubles à traiter ainsi que du poids des malades, et de la voie d'administration.

La dose chez l'homme est variable entre 0,2 et 150 mg de produit, une à trois fois par jour, les doses inférieures étant réservées aux enfants.

Pour la préparation de compositions pharmaceutiques selon la présente invention, les principes actifs (composés de formule (I) et leurs sels pharmaceutiquement acceptables) sont avantageusement formulés en unités de dosage, contenant de 0,2 à 150 mg, de préférence de 0,5 à 50 mg, dudit principe actif, seul ou en mélange avec un excipient pharmaceutique.

A titre d'exemple, lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent de façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, tels que la polyvinylpyrrolidone et similaires, de même qu'avec des édulcorants ou des correcteurs du goût.

Le principe actif peut être formulé également sous forme de microcapsules, éventuellement avec un ou plusieurs supports ou additifs.

Pour une administration sublinguale on peut préparer des micropastilles ou des microcapsules qui, lorsqu'elles sont placées sous la langue, se dissolvent très rapidement. Ces compositions en général contiennent l'ingrédient actif en mélange avec des agents mouillants et/ou des agents de dispersion et éventuellement avec des produits auxiliaires accélérant l'absorption.

Pour une administration transdermique, on peut envisager l'utilisation de matrices de diffusion polymériques pour la libération continue, de préférence sur une période de temps prolongée, du principe actif et l'utilisation du principe actif sous forme de microémulsions formées d'excipients compatibles avec la peau.

Pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol ou le butylèneglycol.

Les compositions pharmaceutiques peuvent être préparées selon les méthodes conventionnelles connues dans la technique (voir par exemple Remington's Pharmaceutical Sciences- Mack Publishing Co.). Des exemples de compositions pharmaceutiques et de méthodes pour leur préparation ont été déjà décrites dans les documents EP 60 176 et EP 101 381.

Les exemples suivants illustrent l'invention.

## PREPARATION

### Composé H :

On chauffe au reflux pendant 20 h un mélange de 9,5 g de 2-chloro-1-(p-tolyl)éthane, 13,9 g de 4-(3-trifluorométhylphényl)-1,2,3,6-tétrahydropyridine base, 150 ml d'éthanol absolu et 7 g de triéthylamine, puis on évapore le solvant sous pression réduite. On reprend le résidu avec 150 ml d'éther éthylique, on lave 3 fois avec 30 ml d'eau, on sèche la phase organique sur du sulfate de sodium et on l'évapore à sec sous pression réduite. On obtient la 1-[2-(4-tolyl)éthylγ-4-(3-trifluorométhylphényl)-1,2,3,6-tétrahydropyridine base sous forme d'une huile. Par traitement avec une solution saturée d'acide chlorhydrique gazeux dans 30 ml d'isopropanol, on obtient le chlorhydrate qui, après recristallisation dans 60 ml d'isopropanol, fond à 263-266°C. Rendement : 4,7 g.

### Exemple 1

On prépare 1 000 comprimés, chacun contenant 2 mg de composé F sous forme de base libre à partir des ingrédients suivants :

| | |
|---|---|
| Composé F | 2,5 g |
| Lactose | 50 g |
| Cellulose microcristalline | 15 g |
| Amidon de maïs séché | 20 g |
| Stéarate de magnésium | 2,5 g. |

On broie l'ingrédient actif jusqu'à une dimension particulaire de 0,4 mm. On le fait alors passer à travers

un tamis de 0,4 mm d'ouverture de maille, et on mélange la matière broyée avec les autres constituants. On comprime le mélange pour former les comprimés.

Exemples 2-4

De la même façon, en remplaçant 2,5 g du composé F par 3,2 g, ou 5,1 g, ou 6,4 g, on prépare 1 000 comprimés contenant chacun respectivement 2,5 mg ou 4 mg, ou 5 mg dudit composé F, sous forme de base libre.

Exemple 5

On prépare 1 000 comprimés contenant chacun 20 mg du composé F, sous forme de base libre à partir des ingrédients suivants :

| | |
|---|---|
| Composé F | 25,5 g |
| Lactose | 79 g |
| Amidon de maïs | 90 g |
| Talc | 5,0 g |
| Stéarate de magnésium | 0,5 g. |

Exemple 6

En opérant comme décrit dans l'exemple 1 mais en utilisant 2,2 g du composé B au lieu de 2,5 g du composé F, on prépare 1 000 comprimés contenant chacun 2 mg, sous forme de base libre, du composé B.

Exemples 7-9

En opérant comme décrit dans l'exemple 6 mais à partir, respectivement, de 2,7 g, 4,4 g, et 5,4 g du composé B, on prépare 1 000 comprimés contenant chacun respectivement 2,5 mg, 4 mg, et 5 mg sous forme de base libre, du composé B.

Exemple 10

On prépare 1 000 capsules, contenant chacune 20 mg, sous forme de base libre, du composé B, à partir des ingrédients suivants :

| | |
|---|---|
| Composé B | 21,9 g |
| Cellulose microcristalline | 77 g |
| Gel de silice amorphe | 1 g. |

On mélange bien les ingrédients ci-dessus et on introduit le mélange ainsi obtenu dans des capsules de gélatine dure ayant la dimension 4.

## Revendications

1. Utilisation d'une trifluorométhylphényltétrahydropyridine de formule (I) :

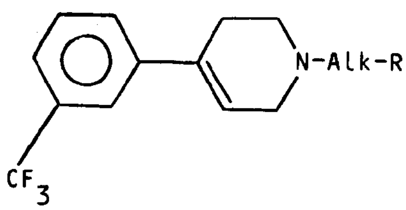

(I)

dans laquelle Alk représente un groupe alkylène à chaîne droite ou ramifiée ayant de 1 à 4 atomes de carbone et R représente un groupe cyano, un groupe acétyle, un groupe cycloalkyle ayant 3 à 7 atomes de carbone, un groupe phényle, un groupe p-tolyle, un groupe pyridyle, un groupe pyridyle 1-oxyde ou un groupe naphtyle, ou d'un de ses sels pharmaceutiquement acceptables, pour la préparation d'un médicament destiné au traitement ou à la prophylaxie des troubles de la motricité intestinale.

2. Utilisation selon la revendication 1, pour la préparation d'un médicament destiné au traitement de la constipation.

3. Utilisation d'une trifluorométhylphényltétrahydropyridine de formule (I) ou d'un de ses sels pharmaceutiquement acceptables selon la revendication 1 ou 2, caractérisée en ce que ladite trifluorométhylphényltétrahydropyridine répond à la formule (I) dans laquelle Alk représente un groupe éthylène, dont 1 ou 2 atomes d'hydrogène peuvent être remplacés par un groupe méthyle.

4. Utilisation selon la revendication 3 du chlorhydrate de 1-[2-(2-naphtyl)éthyl]-4-(3-trifluorométhylphényl)-1,2,3,6-tétrahydropyridine.

5. Utilisation selon la revendication 3 du dichlorhydrate de 1-[2-(3-pyridyl)éthyl]-4-(3-trifluorométhylphényl)-1,2,3,6-tétrahydropyridine monohydraté.

6. Utilisation selon l'une quelconque des revendications 1 à 5, caractérisée en ce que ledit médicament est une composition en unités de dosage.

7. Utilisation selon la revendication 6, caractérisée en ce que l'unité de dosage contient de 0,2 à 150 mg de principe actif.

8. Utilisation selon la revendication 7, caractérisée en ce que l'unité de dosage contient de 0,5 à 50 mg de principe actif.

9. Utilisation selon l'une quelconque des revendications 1 à 8, caractérisée en ce que le médicament est une composition pour l'administration orale.

10. La 1-[2-(4-tolyl)éthyl]-4-(3-trifluorométhylphényl)-1,2,3,6-tétrahydropyridine et ses sels d'addition avec des acides pharmaceutiquement acceptables.

11. Composition pharmaceutique, caractérisée en ce qu'elle contient la 1-[2-(4-tolyl)éthyl]-4-(3-trifluorométhylphényl)-1,2,3,6-tétrahydropyridine ou un de ses sels d'addition avec des acides pharmaceutiquement acceptables.

## Claims

1. Use of a trifluoromethylphenyltetrahydropyridine of formula (I) :

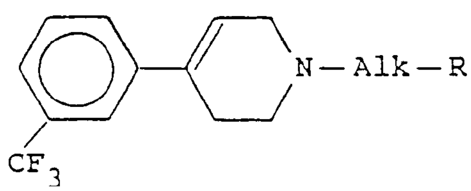

(I)

in which Alk represents a straight or branched alkylene group having from 1 to 4 carbon atoms and R represents a cyano group, an acetyl group, a cycloalkyl group having from 3 to 7 carbon atoms, a phenyl group, a p-tolyl group, a pyridyl group, a pyridyl-1-oxide group or a naphthyl group, or of a pharmaceutically acceptable salt thereof, for the preparation of a drug for the treatment or prophylaxis of intestinal motility disorders.

2. Use according to claim 1, for the preparation of a drug for the treatment of constipation.

3. Use of a trifluoromethylphenyltetrahydropyridine of formula (I) or of a pharmaceutically acceptable salt thereof according to claim 1 or 2, characterized in that said trifluoromethylphenyltetrahydropyridine corresponds to formula (I) in which Alk represents an ethylene group 1 or 2 hydrogen atoms of which can be optionally substituted with a methyl group.

4. Use according to claim 3 of the 1-[2-(2-naphthyl)ethyl]-4-(3-trifluoromethylphenyl)-1,2,3,6-tetrahydropyridine hydrochloride.

5. Use according to claim 3 of the 1-[2-(3-pyridyl)ethyl]-4-(3-trifluoromethylphenyl)-1,2,3,6-tetrahydropyridine monohydrate dihydrochloride.

6. Use according to any one of claims 1 to 5, characterized in that said drug is a composition in dosage unit form.

7. Use according to claim 6, characterized in that the dosage unit form contains from 0.2 to 150 mg of active principle.

8. Use according to claim 7, characterized in that the dosage unit form contains from 0.5 to 50 mg of active principle.

9. Use according to any one of claims 1 to 8, characterized in that the drug is a composition for oral administration.

10. 1-[2-(4-tolyl)ethyl]-4-(3-trifluoromethylphenyl)-1,2,3,6-tetrahydropyridine and its addition salts with pharmaceutically acceptable acids.

11. Pharmaceutical composition, characterized in that it contains the 1-[2-(4-tolyl)ethyl]-4-(3-trifluoromethylphenyl)-1,2,3,6-tetrahydropyridine or an addition salt thereof with pharmaceutically acceptable acids.

## Patentansprüche

1. Verwendung eines Trifluormethylphenyltetrahydropyridins der Formel (I)

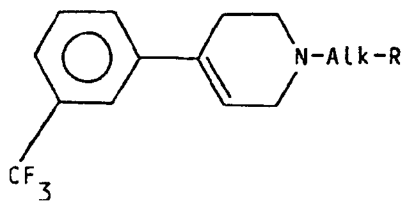

(I)

worin Alk eine geradkettige oder verzweigtkettige Alkylengruppe mit 1-4 Kohlenstoffatomen und R eine Cyanogruppe, eine Acetylgruppe, eine Cycloalkylgruppe mit 3-7 Kohlenstoffatomen, eine Phenylgruppe, eine p-Tolylgruppe, eine Pyridylgruppe, eine Pyridyl-1-Oxidgruppe oder eine Naphtylgruppe bedeuten, oder eines seiner pharmazeutisch verträglichen Salze zur Herstellung eines Medikamentes zur Behandlung oder Prophylaxe von Motilitätsstörungen des Intestinaltrakts.

2. Verwendung nach Anspruch 1 zur Herstellung eines Medikamentes zur Behandlung von Verstopfung.

3. Verwendung eines Trifluormethylphenyltetrahydropyridins der Formel (I) oder eines seiner pharmazeutisch verträglichen Salze nach Anspruch 1 oder 2, dadurch gekennzeichnet, daß das Trifluormethylphenyltetrahydropyridin die Formel (I) besitzt, worin Alk eine Ethylengruppe bedeutet, in der 1 oder 2 Wasserstoffatome durch eine Methylgruppe ersetzt sein können.

4. Verwendung nach Anspruch 3 des 1-[2-(2-Naphtyl)ethyl]-4-(3-trifluormethylphenyl)-1,2,3,6-tetrahydropyridin-Hydrochlorids.

5. Verwendung nach Anspruch 3 des 1-[2-(3-Pyridyl)ethyl]-4-(3-trifluormethylphenyl)-1,2,3,6-tetrahydropyridinmonohydrat-Dihydrochlorids.

6. Verwendung nach einem der Ansprüche 1 bis 5, dadurch gekennzeichnet, daß das Medikament eine Zusammensetzung in Einheitsdosen ist.

7. Verwendung nach Anspruch 6, dadurch gekennzeichnet, daß die Einheitsdosis 0,2 bis 150 mg Wirkstoff enthält.

8. Verwendung nach Anspruch 7, dadurch gekennzeichnet, daß die Einheitsdosis 0,5 bis 50 mg Wirkstoff enthält.

**9.** Verwendung nach einem der Ansprüche 1 bis 8, dadurch gekennzeichnet, daß das Medikament eine Zusammensetzung zur oralen Verabreichung ist.

**10.** 1-[2-(4-Tolyl)ethyl]-4-(3-trifluormethylphenyl)-1,2,3,6-tetrahydropyridin und seine Additionssalze mit pharmazeutisch verträglichen Säuren.

**11.** Pharmazeutische Zusammensetzung, dadurch gekennzeichnet, daß sie 1-[(2-(4-Tolyl)ethyl]-4-(3-trifluormethylphenyl)-1,2,3,6-tetrahydropyridin oder eines seiner Additionssalze mit pharmazeutisch verträglichen Säuren enthält.